(19) Europäisches Patentamt
European Patent Office
Office européen des brevets

(11) Publication number : **0 412 745 B1**

(12) # EUROPEAN PATENT SPECIFICATION

(45) Date of publication of patent specification : **02.03.94 Bulletin 94/09**

(51) Int. Cl.$^5$ : **A61K 7/11**

(21) Application number : **90308612.2**

(22) Date of filing : **06.08.90**

(54) **Hair setting composition.**

The file contains technical information submitted after the application was filed and not included in this specification

(30) Priority : **08.08.89 GB 8918079**

(43) Date of publication of application : **13.02.91 Bulletin 91/07**

(45) Publication of the grant of the patent : **02.03.94 Bulletin 94/09**

(84) Designated Contracting States : **AT BE CH DE DK ES FR GB GR IT LI NL SE**

(56) References cited :
**EP-A- 0 297 455**
**EP-A- 0 403 282**
**DE-A- 3 632 030**
**FR-A- 2 094 109**
**US-A- 3 632 754**
**US-A- 4 765 976**
**US-A- 4 780 310**
**Copy of Applicant's letter dated 01.09.1992 in application no. 90306499.6**

(73) Proprietor : **UNILEVER PLC**
**Unilever House Blackfriars**
**London EC4P 4BQ (GB)**
(84) **GB**
Proprietor : **UNILEVER N.V.**
**Weena 455**
**NL-3013 AL Rotterdam (NL)**
(84) **BE CH DE DK ES FR GR IT LI NL SE AT**

(72) Inventor : **Gallagher, Peter**
**1 Mission Cottage , (Moss Lane)**
**Burscough, Lancashire (GB)**
Inventor : **McGee, Thomas**
**74 Stanley Road**
**Hoylake, Wirral, Merseyside L47 LH2 (GB)**
Inventor : **Khoshdel, Ezat**
**The Nook, Church Lane**
**Neston, Wirral, Merseyside L64 9US (GB)**

(74) Representative : **Tonge, Robert James et al**
**UNILEVER PLC Patent Division Colworth House Sharnbrook**
**Bedford MK44 1LQ (GB)**

EP 0 412 745 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

The present invention relates to a hair setting composition, particularly to a hair setting composition containing a derivative of polyaminoglucose glycan polymer complex (PAGGPC) in solution in a mixture of alcoholic or aqueous/alcoholic solvent and non hydrogen-bonded cosolvent.

### BACKGROUND OF THE INVENTION

Hair setting products are used to retain the hair in a particular style. These products may be in the form of gels, lotions, mousses or sprays and will normally contain film-forming materials as the setting agent. Such known materials include for example certain derivatives of chitosan, as described in US 4 780 310.

Particulary effective setting agents are the water-soluble salts of PAGGPC referred to in our earlier patent application case J3115, filed in EPO as 90306499.6.

PAGGPC may be obtained by treating the mycelia of certain fungal species, such as Aspergillus niger, with strong alkali, as described in for example GB-A-2026516 (Muzzarelli) or US-A-4806474 (Miles). Aspergillus niger is a common waste product in industrial fermentation processes, for example in the production of citric acid.

Polyaminoglucose glycan polymer complex has not been fully characterised, but NMR studies have shown that it is distinct in character from chitosan, and physical comparisons show that films formed by such a complex and by chitosan are different from each other. Polyaminoglucose glycan polymer complex is commercially available, for example as RIOSAN (Trademark) from Meyhall.

In hairspray and mousse preparations a very important criterion for the consumer is that the hairspray is not too wet. The main solvent in hairsprays is generally alcohol, or an aqueous/alcoholic mixture, which does not evaporate quickly enough to avoid imparting a wet feel to the hair. This may be avoided by the use of a more volatile, apolar cosolvent which results in a spray droplet from which most of the solvent evaporates before striking the hair fibre.

The water-soluble salts of PAGGPC referred to above are insoluble in non hydrogen-bonded solvents, and hair setting compositions containing these salts as setting agents are therefore limited in the amount of volatile cosolvent which may be used. Hairsprays of this type are therefore felt to be wet in use.

We have found that certain derivatives of PAGGPC may be incorporated into hairsprays. These derivatives must be soluble in the non hydrogen-bonded cosolvents, so that there is no need to restrict the amount of cosolvent used, and the resulting hair setting compositions are found not to give a wet feeling in use.

### BRIEF SUMMARY OF THE INVENTION

Accordingly the invention provides a hair setting composition comprising a derivative of polyaminoglucose glycan polymer complex which comprises the reaction product of the polymer with an electrophile, an organic or aqueous/organic solvent, and a non hydrogen-bonded cosolvent, said derivative being soluble in a mixture of the solvent and the cosolvent.

### DETAILED DESCRIPTION OF THE INVENTION

PAGGPC may be obtained by treating the mycelia of for example, Aspergillus niger with concentrated alkali to remove impurities and to partially deacetylate the polymer complex. PAGGPC is available for example as RIOSAN which is partially deacetylated.

### Derivatives of PAGGPC

The derivatives of PAGGPC which are used as the film-forming material in the hair setting compositions of the invention comprise the reaction products of PAGGPC with an electrophile.

The electrophile is suitably chosen from epoxide eg. $C_{2-32}$ epoxide, $C_{5-74}$ bisepoxide, $C_{1-30}$ alkyl halide, polyethylene glycol halide, $C_{1-30}$ acyl halide, acid anhydride, $C_{1-30}$ alkyl ester, aryl ester or $C_{1-30}$ aliphatic aldehyde.

Preferred examples of epoxides which may be used as the electrophile include -

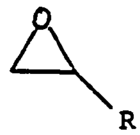

I

where R is H,$C_{1-20}$ saturated or unsaturated alkyl, $C_{2-30}$ ethoxylated alkyl, saturated or unsaturated cycloalkyl e.g. $C_{3-10}$ cycloalkyl, or

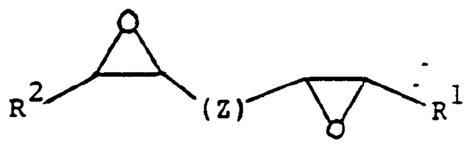

II

where $R^1$,$R^2$ may be the same or different and are H, or $C_{1-20}$ alkyl, Z is aryl, $C_{1-30}$ saturated or unsaturated alkyl or $C_{2-30}$ ethoxylated alkyl, or derivatives thereof.

Examples of suitable halides include 1-bromoethane, 1-bromohexane, 1-chlorododecane, chloropolyethylene glycol, ethanoyl chloride, propionyl chloride, benzoyl chloride and stearoyl chloride.

Suitable acid anhydrides include acetic, succinic, maleic, phthalic and citric anhydride, and suitable esters are ethyl ethanoate, ethyl benzoate and benzoyl benzoate.

The hair setting composition of the invention preferably comprises from 0.01 to 20% by weight of the derivative of PAGGPC and most preferably from 0.1 to 5% by weight.

PAGGPC will normally contain free hydroxyl groups and some acetylated amine groups. Under suitable conditions the electrophile, from those outlined above, will add to the hydroxyl group or groups. However, reaction is often easier, and preferable, if the PAGGPC is deacetylated by treating with strong alkali. The electrophile, under suitable conditions, will then react with both the free amine and the hydroxyl groups, although the electrophile will preferentially attack the amine group(s).

Thus, in accordance with the invention the polyaminoglucose glycan polymer complex is preferably bonded through nitrogen or oxygen to residues of an electrophile which are aliphatic or aromatic groups containing one to thirty two carbon atoms.

It should be noted that for certain electrophiles eg. 1-bromododecane, only a low level of substitution is required to give a derivative of PAGGPC which is soluble in non hydrogen-bonded cosolvents. Higher levels of substitution give rise to derivatives which are not soluble in non hydrogen-bonded cosolvents. Such derivatives would not be suitable for use in the composition of the invention since they would be insoluble in the mixture of organic or aqueous/organic solvent and non hydrogen-bonded cosolvent. The person skilled in the art will readily ascertain the required degree of substitution necessary for any given electrophile.

The use of derivatives having higher levels of substitution is the subject of our copending application J.3119 having the same date as this application.

## Solvent

The hairspray composition of the invention also comprises an organic solvent or an aqueous organic solvent.

Suitable solvents for use in the compositions of the invention include ethanol, isopropanol, methylene chloride, methoxyethanol, 2-ethoxyethanol, and mixtures thereof with water. The composition may comprise more than one such solvent.

The composition will usually comprise from 5 to 90% by weight of solvent and when the composition is in the form of a hairspray in aerosol form, the solvent will preferably form from 20 to 80% by weight of the composition.

## Non hydrogen-bonded cosolvent

The composition of the invention also comprises a non hydrogen-bonded cosolvent. The cosolvent is of a type which quickly evaporates in use, leaving a spray droplet which feels dry. Chlorofluorocarbons, fluorocarbons, hydrocarbons and dimethyl ether and mixtures thereof are particularly suitable cosolvents. Especially suitable are trichlorofluoromethane, dichlorodifluoromethane, 1,2-dichlorotetrafluoroethane, chlorodifluoromethane, 1-chloro,1,1-difluoromethane, 1,1-difluoroethane, butane or propane. Further examples of non hydrogen-bonded solvents may be found in Table II at pages C-696 to C-698 of the Handbook of Chemistry and Physics, 1983-84, 64th Edition, Ed. Robert C Weast, CRC Press, parts A (nonpolar solvents) and B (moderately polar solvents).

The cosolvent is generally present in the composition in an amount of from 5 to 90% by weight, preferably 20 to 80% by weight.

The extent of the dry feeling experienced by the user will depend on the balance between the solvent and the cosolvent used in the composition. Other factors, such as cost, flammability and desired discharge rate must also be taken in to account.

Those skilled in the art will easily ascertain the appropriate balance of solvent and cosolvent for the derivative of PAGGPC. For example, when the solvent is ethanol and the cosolvent is a fluorocarbon, a mixture of ethanol and fluorocarbon in a ratio of 30:70 by weight will give an acceptable, safe spray.

When the cosolvent is hydrocarbon, the flammability of the spray obtained when the product is in the form of a hairspray must also be taken into account. In general, the level of hydrocarbon in an ethanol/hydrocarbon system should be below 50% by weight of the ethanol/hydrocarbon mixture. The level of flammability may be reduced by using a water/ethanol mixture as solvent.

When dimethylether (DME) is used as cosolvent, it may suitably be used in amounts of up to 90% by weight of the solvent/DME mixture.

The ratio of solvent to cosolvent generally lies between 1:9 and 9:1 by weight.

## Other ingredients

The composition of the invention may also include other ingredients such as perfume; alcohol denaturants, for example benzyl diethyl,2,6 -xylyl carbamoyl methyl ammonium benzoate and sucrose octacetate; conditioning agents such as lanolin derivative; alkyl quaternary ammonium compounds such as cetyl trimethyl ammonium bromide; volatile silicones; plasticisers, such as silicone oils or silicone glycol; drag reducing agents such as hydroxypropylcelluloses eg. the range available from Hercules Inc under their trade mark. Klucel or high molecular weight polymers such as PVP-K90 (polyvinyl pyrrolidone having a K value of 90); high molecular weight polymers, such as cationic cellulose resins; other film forming polymers such as polymers of vinyl pyrrolidone and/or vinyl acetate; pH modifiers such as sodium hydroxide, 2 amino-2-methyl-1-propanol, triethanolamine, citric acid, or hydrochloric acid; ingredients to improve combing out such as polydimethyl siloxane-polyoxyalkylene copolymers; corrosion inhibitors, such as triethanolamine salt of alkyenyl amberacide anhydride or disodium dodecyl sulphosucconate; surfactants such as lauryl dimethyl amine oxide.

## Product form

The composition of the invention may be packed in aerosol cans or aerosol PVA packs, or may be in the form of mechanical pumps such as squeeze sprays or pump sprays.

In aerosol hairspray or mousse form, where the cosolvent does not act as a propellant in use, a propellant gas such as air, nitrogen or carbon dioxide may be added. The gas may be situated in the same compartment as the product to be dispensed or in a separate compartment.

The invention is further illustrated by the following Examples.

## EXAMPLES

### Preparation of PAGGPC derivatives

#### Preparation Example A

PAGGPC was N-propoxylated by heating deacetylated PAGGPC in a sealed tube (or an autoclave) with propylene oxide in an aqueous ethanol solvent for 12 hours at 100°C, to give N-hydroxypropyl PAGGPC (A).

#### Preparation Example B

The N-hydroxypropyl PAGGPC derivative obtained in Preparation Example A above was further functionalised by heating at between 60 and 80°C in an autoclave with 1-bromopropane in acetone in the presence of NaOH for 18 hours. O-propyl,N-hydroxypropyl PAGGPC (B) was obtained.

Preparation Example C

Crude, acetylated PAGGPC was heated in dimethyl formamide (DMF) at 100°C for 15 hours in the presence of 1-bromooctane to give O-octyl PAGGPC (C).

Preparation Example D

Deacetylated PAGGPC was heated in DMF with 1-bromohexane at 80°C for 15 hours to give N-hexyl PAGGPC (D).

Preparation Example E

Deacetylated PAGGPC was reacted with succinic anhydride in DMF which contained a small amount of triethylamine, for 18 hours at 85°C, to give N-succinyl PAGGPC (E).

Preparation Example F

PAGGPC was reacted with stearoyl chloride in DMF in the presence of pyridine to give O-stearoyl PAGGPC (F).

In the following examples of hair setting compositions which illustrate the invention,all quantities are % by weight.

| EXAMPLE | | 1 | 2 | 3 |
|---|---|---|---|---|
| PAGGPC (A) | | 3.5 | 3.0 | 1.8 |
| Water | | 17.5 | 10.0 | - |
| DME | | 55.0 | 45.0 | - |
| CFC 11/12[1] (65:35) | | - | - | 60.0 |
| Perfume | | 0.13 | 0.13 | 0.13 |
| Sucrose octacetate | | 0.03 | 0.06 | 0.06 |
| Klucel HF | | 0.06 | 0.06 | - |
| Silicone glycol | | 0.03 | 0.02 | - |
| Ethanol | to | 100 | 100 | 100 |

1 - CFC 11/12 (65:35) is a mixture of 65% by weight trichlorofluoromethane and 35% by weight dichlorodifluoromethane.

| EXAMPLE | | 4 | 5 | 6 |
|---|---|---|---|---|
| PAGGPC (B) | | 2.0 | 1.5 | 3.0 |
| DME | | 40 | 35 | 35 |
| Water | | 20 | 20 | 20 |
| Isopropanol | | 10 | 15 | - |
| Sucrose octacetate | | 0.1 | 0.1 | 0.1 |
| Perfume | | 0.2 | 0.2 | 0.2 |
| Klucel HF | | 0.05 | 0.04 | 0.04 |
| Silicone glycol | | 0.03 | 0.03 | 0.04 |
| Ethanol | to | 100 | 100 | 100 |

| EXAMPLE | | 7 | 8 | 9 |
|---|---|---|---|---|
| PAGGPC (C) | | 3.0 | 3.0 | 2.0 |
| CFC F114 [2] | | 50 | - | - |
| Hydrocarbon (CAP 30) | | - | 45 | 30 |
| Methylene chloride | | - | 2 | 5 |
| Water | | - | - | 10 |
| Compressed air | | - | - | 0.5 |
| Isopropanol | | 10 | 15 | 10 |
| Sucrose octaacetate | | 0.1 | 0.1 | 0.1 |
| Perfume | | 0.2 | 0.2 | 0.2 |
| PVP-K90 | | 0.04 | 0.05 | 0.04 |
| Ethanol | to | 100 | 100 | 100 |

2 - CFC F114 is 1,2-dichlorotetrafluoroethane

| EXAMPLE | | 10 | 11 | 12 |
|---|---|---|---|---|
| PAGGPC (D) | | 1.5 | 1.0 | 3.0 |
| DME | | 40 | 40 | 45 |
| Water | | 15 | 15 | 20 |
| Isopropanol | | 20 | 20 | 20 |
| Sucrose octacetate | | 0.1 | 0.1 | 0.1 |
| Perfume | | 0.2 | 0.2 | 0.2 |
| Klucel HF | | 0.06 | 0.03 | 0.03 |
| Silicone glycol | | 0.05 | 0.02 | 0.02 |
| Ethanol | to | 100 | 100 | 100 |

| EXAMPLE | | 13 | 14 | 15 |
|---|---|---|---|---|
| PAGGPC (E) | | 1.5 | 1.5 | 3.0 |
| CFC F114 | | 35 | 40 | 40 |
| Methylene chloride | | 4 | 4 | 2 |
| Nitrogen | | 0.5 | - | 0.1 |
| Carbon dioxide | | - | 4.5 | - |
| Isopropanol | | 15 | 5 | - |
| Perfume | | 0.2 | 0.2 | 0.2 |
| PVP-K90 | | 0.04 | 0.05 | 0.05 |
| Ethanol | to | 100 | 100 | 100 |

| EXAMPLE | 16 | 17 | 18 | 19 |
|---|---|---|---|---|
| PAGGPC (F) | 4.0 | 3.5 | 2.5 | 1.0 |
| CFC 142 B [3] | 60 | - | - | 40 |
| Hydrocarbon (CAP 30) | - | 40 | 5 | - |
| Methylene chloride | 5 | 5 | 5 | - |
| Carbon dioxide | - | - | 1 | 1 |
| Isopropanol | 10 | 15 | 15 | 10 |
| Sucrose octacetate | 0.1 | 0.1 | 0.1 | 0.1 |
| Perfume | 0.3 | 0.3 | 0.3 | 0.3 |
| Klucel HF | 0.03 | 0.04 | 0.04 | 0.05 |
| Ethanol to | 100 | 100 | 100 | 100 |

3 - CFC 142 B is 1-chloro,1,1-difluoromethane.

Example 20

The following is an example of a mousse according to the invention:

| | % w/w |
|---|---|
| PAGGPC (B) | 2.0 |
| Ethanol | 15 |
| DME | 7 |
| Empigen OB [4] | 0.03 |
| Silicone glycol | 0.05 |
| Perfume | q.s. |
| Arquad 16/50 [5] | 0.08 |
| Water | to 100 |

4 - Empigen OB is lauryl dimethylamine oxide

5 - Arquad 16/50 is a mixture of trimethylammonium chloride and isopropyl alcohol

## Claims

1. A hair setting composition comprising a derivative of polyaminoglucose glycan polymer complex which comprises the reaction product of the polymer with an electrophile, an organic or aqueous organic solvent, and a non hydrogen-bonded cosolvent, where said derivative is dissolved in a mixture of the solvent and

the cosolvent.

2. A hair setting composition as claimed in Claim 1, wherein the polyaminoglucose glycan polymer complex is bonded through nitrogen or oxygen to residues of the electrophile which are aliphatic or aromatic groups containing one to thirty two carbon atoms.

3. A hair setting composition as claimed in Claim 1 or claim 2, wherein the electrophile is chosen from epoxide, $C_{5-74}$ bisepoxide, $C_{1-30}$ alkyl halide, polyethylene glycol halide, $C_{1-30}$ acyl halide, acid anhydride, $C_{1-30}$ alkyl esters, aryl esters or $C_{1-30}$ aliphatic aldehyde.

4. A hair setting composition as claimed in any one of Claims 1 to 3, wherein the epoxide is chosen from

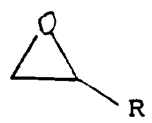

I

where R is H, $C_{1-20}$ saturated or unsaturated alkyl, $C_{2-30}$ ethoxylated alkyl, saturated or unsaturated cycloalkyl, or

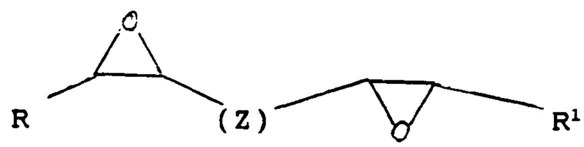

II

where R, $R^1$ may be the same or different and are H, or $C_{1-20}$ alkyl, and Z is aryl, $C_{1-30}$ saturated or unsaturated alkyl or $C_{2-30}$ ethoxylated alkyl, or derivatives thereof.

5. A hair setting composition as claimed in any one of Claims 1 to 3, wherein the acid anhydride is chosen from acetic, succincic, maleic, phthalic or citric anhydride.

6. A hair setting composition as claimed in any preceding claim, wherein the derivative of polyaminoglucose glycan polymer complex is present in an amount of from 0.01 to 20% by weight.

7. A hair setting composition as claimed in Claim 6, wherein the derivative of polyaminoglucose glycan polymer complex is present is an amount of from 0.1 to 5% by weight.

8. A hair setting composition as claimed in any preceding claim, wherein the solvent is chosen from ethanol, isopropanol, methylene chloride, methoxyethanol and 2-ethoxyethanol, mixtures thereof, and mixtures thereof with water.

9. A hair setting composition as claimed in any preceding claim, wherein the non hydrogen-bonded cosolvent is chosen from chlorofluorocarbons, fluorocarbons, hydrocarbons and dimethylether.

## Patentansprüche

1. Haarfestigerzusammensetzung umfassend ein Derivat des Polyaminoglucose-Glykanpolymerkomplexes, der das Reaktionsprodukt des Polymers mit einem Elektrophil umfaßt, ein organisches oder wäßriges organisches Lösungsmittel und ein nicht durch Wasserstoffbrücken gebundenes Co-Lösungsmittel, wobei besagtes Derivat in einer Mischung des Lösungsmittels und des Co-Lösungsmittels aufgelöst ist.

2. Haarfestigerzusammensetzung gemäß Anspruch 1, wobei der Polyaminoglucose-Glykanpolymerkomplex durch Stickstoff oder Sauerstoff an Reste des Elektrophils gebunden ist, die aliphatische oder aromatische Gruppen sind, die 1 bis 32 Kohlenstoffatome enthalten.

3. Haarfestigerzusammensetzung gemäß Anspruch 1 oder 2, wobei das Elektrophil ausgewählt ist von Epoxid, $C_{5-74}$ Bisepoxid, $C_{1-30}$ Alkylhalogenid, Polyethylen-glycolhalogenid, $C_{1-30}$ Acylhalogenid, Säureanhydrid, $C_{1-30}$ Alkylestern, Arylestern oder $C_{1-30}$ aliphatischem Aldehyd.

4. Haarfestigerzusammensetzung gemäß einem der Ansprüche 1 bis 3, wobei das Epoxid ausgewählt ist von

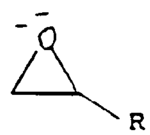

I

wobei R Wasserstoff, $C_{1-20}$ gesättigtes oder ungesättigtes Alkyl, $C_{2-30}$ ethoxyliertes Alkyl, gesättigtes oder ungesättigtes Cycloalkyl ist, oder

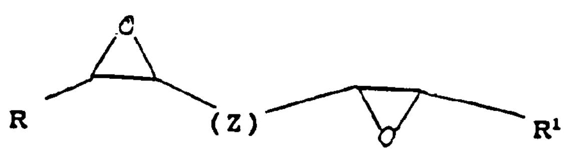

II

wobei R, $R^1$ gleich oder verschieden sein können und Wasserstoff oder $C_{1-20}$ Alkyl sind, und Z Aryl, $C_{1-30}$ gesättigtes oder ungesättigtes Alkyl oder $C_{2-30}$ ethoxyliertes Alkyl oder Derivate davon ist.

5. Haarfestigerzusammensetzung gemäß einem der Ansprüche 1 bis 3, wobei das Säureanhydrid ausgewählt ist von Acetanhydrid, Bernsteinsäureanhydrid, Maleinsäureanhydrid, Phthalsäureanhydrid oder Zitronensäureanhydrid.

6. Haarfestigerzusammensetzung gemäß einem der vorangehenden Ansprüche, wobei das Derivat des Polyaminoglucose-Glykanpolymerkomplexes in einer Menge von 0,01 bis 20 Gewicht-% vorhanden ist.

7. Haarfestigerzusammensetzung gemäß Anspruch 6, wobei das Derivat des Polyaminoglucose-Glykanpolymerkomplexes in einer Menge von 0,1 bis 5 Gewicht-% vorhanden ist.

8. Haarfestigerzusammensetzung gemäß einem der vorangehenden Ansprüche, wobei das Lösungsmittel ausgewählt ist von Ethanol, Isopropanol, Methylenchlorid, Methoxyethanol und 2-Ethoxyethanol, Mischungen davon und Mischungen davon mit Wasser.

9. Haarfestigerzusammensetzung gemäß einem der vorangehenden Ansprüche, wobei das nicht durch Wasserstoffbrücken gebundene Co-Lösungsmittel ausgewählt ist von Fluorchlorkohlenwasserstoffen, Fluorkohlenstoffen, Kohlenwasserstoffen und Dimethylether.

## Revendications

1. Composition de fixation de cheveux comprenant un dérivé d'un complexe polymère polyaminoglucose/glycane qui comprend le produit de réaction du polymère avec un électrophile, un solvant organique ou organique aqueux et un co-solvant non lié par l'hydrogène, ledit dérivé étant dissous dans un mélange du solvant et du co-solvant.

2. Composition de fixation de cheveux selon la revendication 1, dans laquelle le complexe polymère polyaminoglucose/glycane est lié par l'azote ou l'oxygène à des résidus de l'électrophile qui sont des groupes aliphatiques ou aromatiques contenant de 1 à 32 atomes de carbone.

3. Composition de fixation de cheveux selon la revendication 1 ou 2, dans laquelle l'électrophile est choisi parmi un époxyde, un bisépoxyde en $C_{5-74}$, un halogénure d'alkyle en $C_{1-30}$, un halogénure de polyéthylèneglycol, un halogénure d'acyle en $C_{1-30}$, un anhydride d'acide, des esters alkyliques en $C_{1-30}$, des esters aryliques ou un aldéhyde aliphatique en $C_{1-30}$.

4. Composition de fixation de cheveux selon l'une quelconque des revendications 1 à 3, dans laquelle l'époxyde est choisi parmi les groupes :

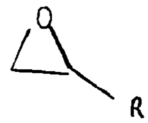

I

dans lesquels R représente H, un radical alkyle saturé ou insaturé en $C_{1-20}$, un radical alkyle éthoxylé en $C_{2-30}$, un radical cycloalkyle saturé ou insaturé ou un groupe

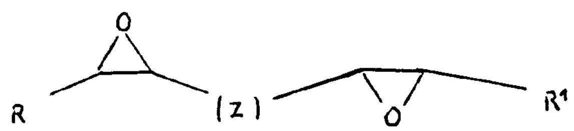

II

dans lequel R et $R^1$ peuvent être identiques ou différents et représentent H ou un radical alkyle en $C_{1-20}$, et Z est un radical aryle, alkyle saturé ou insaturé en $C_{1-30}$ ou alkyle éthoxylé en $C_{2-30}$, ou des dérivés de ceux-ci.

5. Composition de fixation de cheveux selon l'une quelconque des revendications 1 à 3, dans laquelle l'anhydride d'acide est choisi parmi les anhydrides acétique, succinique, maléique, phtalique ou citrique.

6. Composition de fixation de cheveux selon l'une quelconque des revendications précédentes, dans laquelle le dérivé du complexe polymère polyaminoglucose/glycane est présent en une quantité de 0,01 à 20% en poids.

7. Composition de fixation de cheveux selon la revendication 6, dans laquelle le dérivé de complexe polymère polyaminoglucose/glycane est présent à raison de 0,1 à 5% en poids.

8. Composition de fixation de cheveux selon l'une quelconque des revendications précédentes, dans laquelle le solvant est choisi parmi l'éthanol, l'isopropanol, le chlorure de méthylène, le méthoxyéthanol et le 2-éthoxyéthanol, leurs mélanges et leurs mélanges avec de l'eau.

9. Composition de fixation de cheveux selon l'une quelconque des revendications précédentes, dans laquelle le co-solvant non lié à l'hydrogène est un chlorofluorocarbone, un fluorocarbone, un hydrocarbure ou l'éther diméthylique.